# EUROPEAN PATENT APPLICATION

(11) **EP 3 950 651 A1**
(43) Date of publication of application: **09.02.2022**
(21) Application number: 20778829.0
(22) Date of filing: 27.03.2020
(51) Int. Cl.: C07C 19/05, C07C 21/073, C07C 17/383

(54) **AZEOTROPIC OR AZEOTROPIC-LIKE COMPOSITION COMPRISING HYDROGEN FLUORIDE AND 1,1,2-TRICHLOROETHANE, TRANS-1,2-DICHLOROETHYLENE OR CIS-1,2-DICHLOROETHYLENE**

(30) Priority: 28.03.2019 JP 2019064663
(71) Applicant: DAIKIN INDUSTRIES, LTD., Osaka-shi, Osaka 530-8323 (JP)
(72) Inventor: TAKAHASHI, Kazuhiro, Osaka-shi, Osaka 530-8323 (JP)
(74) Representative: Hoffmann Eitle
(86) International application number: PCT/JP2020/014074
(87) International publication number: WO 2020/196843

(57) **Abstract**

An object of the present invention is to provide a novel azeoropic or azeotrope-like composition that is effective for the separation of HF from 1,1,2-trichloroethane (HCC-140), trans-1,2-dichloroethylene (HCO-1130(E)), or cis-1,2-dichloroethylene (HCO-1130(Z)). The object can be achieved by an azeotropic or azeotrope-like composition comprising HCC-140, HCO-1130(E), or HCO-1130(Z), and hydrogen fluoride.

## Description

### Technical Field

The present invention relates to an azeotropic or azeotrope-like composition comprising 1,1,2-trichloroethane, trans-1,2-dichloroethylene, or cis-1,2-dichloroethylene, and hydrogen fluoride.

### Background Art

Refrigerants comprising trans-1,2-difluoroethylene and cis-1,2-difluoroethylene (respectively referred to as "HFO-1132(E)" and "HFO-1132(Z)"), which are HFOs (hydrofluoroolefins), i.e., HFCs of olefins with low global warming potential, are very promising alternative substances to refrigerants such as R-410A, HFC-32, HFC-134a, and the like, which have been used in air conditioners (Patent Literature 1). These fluoroalkenes are often produced by reacting haloalkanes or haloalkenes having the corresponding carbon number as raw materials with hydrogen fluoride by a gas-phase or liquid-phase fluorination reaction in the presence or absence of a catalyst. In this process, the conversion rate of the raw materials (haloalkanes or haloalkenes and hydrogen fluoride) is not always 100%, and it is necessary to separate the reaction product gas from the raw materials.

As a method of producing E-1,2-difluoroethylene (HFO-1132(E)), dehydrogen fluoride reaction of 1,1,2-trifluoroethane (HFC-143) is a most reasonable method to obtain HFO-1132(E). As a method of producing HFC-143, a method in which a fluorination reaction is performed using 1,1,2-trichloroethane (HCC-140), trans-1,2-dichloroethylene (HCO-1130(E)), or cis-1,2-dichloroethylene (HCO-1130(Z)) as a raw material in the presence or absence of a catalyst, under gas phase or liquid phase conditions, is a promising method in terms of yield.

### Citation List

### Patent Literature

PTL 1: WO2012/157765

### Summary of Invention

### Technical Problem

An object of the present disclosure is to provide a novel azeoropic or azeotrope-like composition that is effective for the separation of HF and HCC-140, HCO-1130(E), or HCO-1130(Z), which is a raw material used in the production of a reaction product comprising HFC-143, HFO-1132(E), and HFO-1132(Z), and their intermediates, such as 1,2-dichloro-1 fluoroethane (HCFC-141), trans-1-chloro-2-fluoroethylene (HCFO-1131(E)), cis-1-chloro-2-fluoroethylene (HCFO-1131(Z)), or 1-chloro-1,2-difluoroethane (HCFC-142).

### Solution to Problem

1. An azeotropic or azeotrope-like composition comprising 1,1,2-trichloroethane (HCC-140) and hydrogen fluoride.
2. The azeotropic or azeotrope-like composition according to Item 1, wherein the HCC-140 is present in an amount of more than 10 mass% and 99 mass% or less based on the entire composition.
3. An azeotropic or azeotrope-like composition comprising trans-1,2-dichloroethylene (HCO-1130(E)) and hydrogen fluoride.
4. The azeotropic or azeotrope-like composition according to Item 3, wherein the HCO-1130(E) is present in an amount of more than 10 mass% and 99 mass% or less based on the entire composition.
5. An azeotropic or azeotrope-like composition comprising cis-1,2-dichloroethylene (HCO-1130(Z)) and hydrogen fluoride.
6. The azeotropic or azeotrope-like composition according to Item 5, wherein the HCO-1130(Z) is present in an amount of more than 15 mass% and 99 mass% or less based on the entire composition.
7. A method of separating a mixture comprising HCC-140 and hydrogen fluoride, comprising the steps of:
   (a) supplying a composition comprising HCC-140 and hydrogen fluoride to a first distillation column;
   (b) extracting, as a first distillate, an azeotropic composition comprising HCC-140 and hydrogen fluoride, and extracting, as a bottom composition of the first distillation column, a composition that is more enriched in either HCC-140 or hydrogen fluoride, in terms of the concentration, than the composition supplied in (a); and optionally
   (c) supplying the first distillate to a second distillation column having operation conditions different from the operation conditions of the first distillation column, followed by distillation.
8. A method of separating a mixture comprising HCO-1130(E) and hydrogen fluoride, comprising the steps of:
   (d) supplying a composition comprising HCO-1130(E) and hydrogen fluoride to a first distillation column;
   (e) extracting, as a first distillate, an azeotropic composition comprising HCO-1130(E) and hydrogen fluoride, and extracting, as a bottom composition of the first distillation column, a composition that is more enriched in either HCO-1130(E) or hydrogen fluoride, in terms of the concentration, than the composition supplied in (d); and optionally
   (f) supplying the first distillate to a second distillation column having operation conditions different from the operation conditions of the first distillation column, followed by distillation.
9. A method of separating a mixture comprising HCO-1130(Z) and hydrogen fluoride, comprising the steps of:
   (g) supplying a composition comprising HCO-1130(Z) and hydrogen fluoride to a first distillation column;
   (h) extracting, as a first distillate, an azeotropic composition comprising HCO-1130(Z) and hydrogen fluoride, and extracting, as a bottom composition of the first distillation column, a composition that is more enriched in either HCO-1130(Z) or hydrogen fluoride, in terms of the concentration, than the composition supplied in (g); and optionally
   (i) supplying the first distillate to a second distillation column having operation conditions different from the operation conditions of the first distillation column, followed by distillation.
10. The separation method according to Items 7 to 9, wherein the distillation is performed in a pressure range of 0.05 MPa to 1 MPa.

### Advantageous Effects of Invention

The present disclosure provides a novel azeotropic or azeotrope-like composition.

### Brief Description of Drawings

Fig. 1 shows an example of the process of separation by distillation using an azeotropic composition.
Fig. 2 shows an example of the process of separation by distillation using an azeotropic composition.
Fig. 3 shows an example of the process of separation by distillation using an azeotropic composition.

### Description of Embodiments

### Definition of Terms

In the present specification, the term "azeotrope-like composition" refers to a composition that can be handled in substantially the same manner as azeotropic compositions. Specifically, in the present specification, the term "azeotrope-like composition" means a mixture composed of two or more substances that behave substantially as a single substance with a constant boiling point, or substantially a constant boiling point. A feature of an azeotrope-like composition is that vapor generated by evaporating or distilling the composition in liquid form has a formulation substantially unchanged from the formulation of the liquid. Specifically, in the present specification, a mixture that can be boiled, distilled, or refluxed without a substantial compositional change is referred to as an "azeotrope-like composition." Specifically, a composition having a difference between the bubble-point pressure and the dew-point pressure of 3% or less (based on the bubble-point pressure) at a specific temperature is defined as an azeotrope-like composition in the present disclosure.

In the present specification, an azeotropic composition and an azeotrope-like composition in which the liquid phase separates into two liquid phases are respectively referred to as a "heterophase azeotropic composition" and a "heterophase azeotrope-like composition."

The unit for the pressure described in the present specification is absolute pressure (MPa). Specifically, the specification describes atmospheric pressure as being about 0.1013 MPa.

The present inventors focused on the fact that in the conventional production methods of HFC-143, the raw materials used are not all converted into the target products, and need to be separated, collected, and recycled by a specific method. This is because these raw materials, if not collected, will be wasted, which leads to increased costs.

The inventors found that combinations of the specific components contained in these raw materials form azeotropic or azeotrope-like compositions, and further found that these compositions are useful in separation based on a method such as distillation, extraction, or liquid-liquid separation. The present invention was thus completed.

### 1. Composition 1

Composition 1 is an azeotropic or azeotrope-like composition comprising HCC-140 and hydrogen fluoride (HF) in an amount efficient for forming an azeotropic or azeotrope-like composition.

Composition 1 preferably comprises HCC-140 in an amount of more than 10 mass% and 99 mass% or less based on the entire composition.

Composition 1 may further comprise an additional compound in addition to HCC-140 and hydrogen fluoride. The total content and kind of the additional compound can be suitably selected as long as the additional compound does not interfere with composition 1 comprising a mixture of HCC-140 and hydrogen fluoride, which is an azeotropic or azeotrope-like composition, becoming an azeotropic or azeotrope-like composition.

The additional compound is preferably contained in a total amount of more than 0 mass% and 1 mass% or less, more preferably more than 0 mass% and 0.5 mass% or less, and even more preferably more than 0 mass% and 0.1 mass% or less, based on the entire composition 1.

The additional compound is not particularly limited and can be broadly selected as long as it does not interfere with composition 1 becoming an azeotropic or azeotrope-like composition. The additional compounds may be used singly, or in a combination of two or more.

Examples of the additional compound include 1-chloro-1,2,2-trifluoroethane (HCFC-133), 1-chloro-1,1,2-trifluoroethane (HCFC-133b), 2-chloro-1,1,1-trifluoroethane (HCFC-133a), 1,1-dichloro-2,2,2-trifluoroethane (HCFC-123), 1,1-difluoroethane (HFC-152a), 1,2-difluoroethane (HFC-152), fluoroethane (HFC-161), 1,1,1-trifluoroethane (HFC-143a), 1,1,1,2-tetrafluoroethane (HFC-134a), difluoromethane (HFC-32), pentafluoroethane (HFC-125), fluoromethane (HFC-41), chlorodifluoromethane (HFC-22), ethylene, propylene, acetylene, 1-chloro-1,2-difluoroethane (HCFC-142a), 2,3,3,3-tetrafluoropropene (HFO-1234yf), 1,3,3,3-tetrafluoropropene (HFO-1234ze), 1,2,3,3,3-pentafluoropropene (HFO-1225ye), fluoroethylene (HCFO-1141), 1,1-difluoroethylene (HFO-1132a), 1-chloro-2,2-difluoroethylene (HCFO-1122), and 1-chloro-1,2-difluoroethylene (HCFO-1122a), and the like.

Composition 1 can serve as an important composition when azeotropic distillation of a mixture of HCC-140 and an additional compound is performed to separate the additional compound from HCC-140.

Azeotropic distillation refers to a method of concentrating or separating a target product by operating a distillation column under such conditions that an azeotropic or azeotrope-like composition is separated. In some cases, azeotropic distillation can distill only the component to be separated, but in other cases, azeotropic distillation may occur only when another component that forms an azeotropic mixture with one or more components to be separated is externally added. In a narrow sense, only the latter is called "azeotropic distillation." For example, an additional compound can be separated from HCC-140 by extracting an azeotropic or azeotrope-like composition comprising HCC-140 and the additional compound from a composition comprising at least HCC-140 and the additional compound by azeotropic distillation.

### 2. Composition 2

Composition 2 is an azeotropic or azeotrope-like composition consisting essentially of HCO-1130(E) and hydrogen fluoride. Specifically, it is an azeotropic or azeotrope-like composition comprising HCO-1130(E) and hydrogen fluoride in an amount efficient for forming an azeotropic or azeotrope-like composition.

Composition 2 preferably comprises HCC-1130(E) in an amount of more than 10 mass% and less than 99 mass%, and more preferably more than 25 mass% and less than 75 mass% based on the entire composition.

Composition 2 may further comprise an additional compound in addition to HCO-1130(E) and hydrogen fluoride. The total content and kind of the additional compound can be suitably selected as long as the additional compound does not interfere with composition 2 comprising a mixture of HCO-1130 (E) and hydrogen fluoride, which is an azeotropic or azeotrope-like composition, becoming an azeotropic or azeotrope-like composition.

The additional compound is preferably contained in a total amount of more than 0 mass% and 1 mass% or less, more preferably more than 0 mass% and 0.5 mass% or less, and even more preferably more than 0 mass% and 0.1 mass% or less, based on the entire composition 2.

The additional compound is not particularly limited and can be broadly selected as long as it does not interfere with composition 2 becoming an azeotropic or azeotrope-like composition. The additional compounds may be used singly, or in a combination of two or more.

Examples of the additional compound include 1-chloro-1,2,2-trifluoroethane (HCFC-133), 1-chloro-1,1,2-trifluoroethane (HCFC-133b), 2-chloro-1,1,1-trifluoroethane (HCFC-133a), 1,2-difluoroethane (HFC-152), fluoroethane (HFC-161), 1,1,1-trifluoroethane (HFC-143a), 1,1,1,2-tetrafluoroethane (HFC-134a), difluoromethane (HFC-32), pentafluoroethane (HFC-125), fluoromethane (HFC-41), chlorodifluoromethane (HFC-22), ethylene, propylene, acetylene, 1-chloro-1,2-difluoroethane (HCFC-142a), 2,3,3,3-tetrafluoropropene (HFO-1234yf), 1,3,3,3-tetrafluoropropene (HFO-1234ze), 1,2,3,3,3-pentafluoropropene (HFO-1225ye), fluoroethylene (HCFO-1141), 1,1-difluoroethylene (HFO-1132a), 1-chloro-2,2-difluoroethylene (HCFO-1122), and 1-chloro-1,2-difluoroethylene (HCFO-1122a), and the like.

Composition 2 can serve as an important composition when azeotropic distillation of a mixture of HCO-1130(E) and an additional compound is performed to separate the additional compound from HCO-1130(E).

For example, an additional compound can be separated from HCO-1130(E) by extracting an azeotropic or azeotrope-like composition comprising HCO-1130(E) and the additional compound from a composition comprising at least HCO-1130(E) and the additional compound by azeotropic distillation.

### 3. Composition 3

Composition 3 is an azeotropic or azeotrope-like composition consisting essentially of HCO-1130(Z) and hydrogen fluoride. Specifically, composition 3 is an azeotropic or azeotrope-like composition comprising HCO-1130(Z) and hydrogen fluoride in an amount efficient for forming an azeotropic or azeotrope-like composition.

Composition 3 preferably comprises HCO-1130(Z) in an amount of more than 15 mass% and 99 mass% or less, and more preferably more than 20 mass% and less than 65 mass% based on the entire composition.

Composition 3 may further comprise an additional compound in addition to HCO-1130(Z) and hydrogen fluoride. The total content and kind of the additional compound can be suitably selected as long as the additional compound does not interfere with composition 3 comprising a mixture of HCO-1130(Z) and hydrogen fluoride, which is an azeotropic or azeotrope-like composition, becoming an azeotropic or azeotrope-like composition.

The additional compound is preferably contained in a total amount of more than 0 mass% and 1 mass% or less, more preferably more than 0 mass% and 0.5 mass% or less, and even more preferably more than 0 mass% and 0.1 mass% or less, based on the entire composition 3.

The additional compound is not particularly limited and can be broadly selected as long as it does not interfere with composition 3 becoming an azeotropic or azeotrope-like composition. The additional compounds may be used singly, or in a combination of two or more.

Examples of the additional compound include 1-chloro-1,2,2-trifluoroethane (HCFC-133), 1-chloro-1,1,2-trifluoroethane (HCFC-133b), 2-chloro-1,1,1-trifluoroethane (HCFC-133a), 1,1-dichloro-2,2,2-trifluoroethane (HCFC-123), 1,1-difluoroethane (HFC-152a), 1,2-difluoroethane (HFC-152), fluoroethane (HFC-161), 1,1,1-trifluoroethane (HFC-143a), 1,1,1,2-tetrafluoroethane (HFC-134a), difluoromethane (HFC-32), pentafluoroethane (HFC-125), fluoromethane (HFC-41), chlorodifluoromethane (HFC-22), ethylene, propylene, acetylene, 1-chloro-1,2-difluoroethane (HCFC-142a), 2,3,3,3-tetrafluoropropene (HFO-1234yf), 1,3,3,3-tetrafluoropropene (HFO-1234ze), 1,2,3,3,3-pentafluoropropene (HFO-1225ye), fluoroethylene (HCFO-1141), 1,1-difluoroethylene (HFO-1132a), 1-chloro-2,2-difluoroethylene (HCFO-1122), and 1-chloro-1,2-difluoroethylene (HCFO-1122a), and the like.

Composition 3 can serve as an important composition when azeotropic distillation of a mixture of HCO-1130(Z) and an additional compound is performed to separate the additional compound from HCO-1130(Z).

For example, an additional compound can be separated from HCO-1130(Z) by extracting an azeotropic or azeotrope-like composition comprising HCO-1130(Z) and the additional compound from a composition comprising at least HCO-1130(Z) and the additional compound by azeotropic distillation.

### 2. Separation Method

The present disclosure also explains a method of separating these components using the composition described above.

For example, by extracting an azeotropic or azeotrope-like composition comprising HCC-140, HCO-1130(E), or HCO-1130(Z), and hydrogen fluoride from the composition at least comprising HCC-140, HCO-1130(E), or HCO-1130(Z), and hydrogen fluoride and an additional component by azeotropic distillation, the additional component can be separated from HCC-140, HCO-1130(E), or HCO-1130(Z).

A simple method of separating HF from HCC-140, HCO-1130(E), or HCO-1130(Z) is a method in which HF is absorbed in water by washing with water, thus collecting HF. However, this method is very inefficient because in many cases water that absorbs hydrogen fluoride becomes hydrogen fluoride acid, which must be disposed as waste, leading to increased costs.

According to the separation method of the present disclosure, the above problems can be solved.

The separation method of the mixture comprising HCC-140 and hydrogen fluoride according to the present disclosure specifically includes
(a) supplying a composition comprising HCC-140 and hydrogen fluoride to a first distillation column;
(b) extracting, as a first distillate, an azeotropic composition comprising HCC-140 and hydrogen fluoride, and extracting, as a bottom composition of the first distillation column, a composition that is more enriched in either HCC-140 or hydrogen fluoride, in terms of the concentration, than the composition supplied in (a); and optionally
(c) supplying the first distillate to a second distillation column having operation conditions different from the operation conditions of the first distillation column, followed by distillation.

In the separation method, the composition comprising HCC-140 and hydrogen fluoride as a starting composition for use in step (a) may be a composition consisting of HCC-140 and hydrogen fluoride, or may be a composition further comprising other components in addition to HCC-140 and hydrogen fluoride.

In the separation method described above, step (c) is not an essential step, and is optional.

The separation method of a mixture comprising HCC-140 and hydrogen fluoride according to the present disclosure may be a method that consists of steps (a) to (C) above, or a method that further comprises other steps in addition to steps (a) to (c) above.

The operating conditions for each of the first and second distillation columns can be appropriately set.

The separation method of the mixture comprising HCO-1130(E) and hydrogen fluoride according to the present disclosure specifically includes
(d) supplying a composition comprising HCO-1130(E) and hydrogen fluoride to a first distillation column;
(e) extracting, as a first distillate, an azeotropic composition comprising HCO-1130(E) and hydrogen fluoride, and extracting, as a bottom composition of the first distillation column, a composition that is more enriched in either HCO-1130(E) or hydrogen fluoride, in terms of the concentration, than the composition supplied in (d); and optionally
(f) supplying the first distillate to a second distillation column having operation conditions different from the operation conditions of the first distillation column, followed by distillation.

In the separation method, the composition comprising HCO-1130(E) and hydrogen fluoride as a starting composition for use in step (d) may be a composition consisting of HCO-1130(E) and hydrogen fluoride, or may be a composition further comprising other components in addition to HCO-1130(E) and hydrogen fluoride.

In the separation method described above, step (f) is not an essential step, and is optional.

The separation method of a mixture comprising HCO-1130(Z) and hydrogen fluoride according to the present disclosure may be a method that consists of steps (d) to (f) above, or a method that further comprises other steps in addition to steps (d) to (f) above.

The operating conditions for each of the first and second distillation columns can be appropriately set.

The separation method of the mixture comprising HCO-1130(Z) and hydrogen fluoride according to the present disclosure specifically includes
(g) supplying a composition comprising HCO-1130(Z) and hydrogen fluoride to a first distillation column;
(h) extracting, as a first distillate, an azeotropic composition comprising HCO-1130(Z) and hydrogen fluoride, and extracting, as a bottom composition of the first distillation column, a composition that is more enriched in either HCO-1130(Z) or hydrogen fluoride, in terms of the concentration, than the composition supplied in (g); and optionally
(i) supplying the first distillate to a second distillation column having operation conditions different from the operation conditions of the first distillation column, followed by distillation.

In the separation method, the composition comprising HCO-1130(Z) and hydrogen fluoride as a starting composition for use in step (g) may be a composition consisting of HCO-1130(Z) and hydrogen fluoride, or may be a composition further comprising other components in addition to HCO-1130(Z) and hydrogen fluoride.

In the separation method described above, step (h) is not an essential step, and is optional.

The separation method of a mixture comprising HCO-1130(Z) and hydrogen fluoride according to the present disclosure may be a method that consists of steps (g) to (i) above, or a method that further comprises other steps in addition to steps (g) to (i) above.

The operating conditions for each of the first and second distillation columns can be appropriately set.

The embodiments are described above; however, it will be understood that various changes in forms and details can be made without departing from the spirit and scope of the claims.

### Examples

The present disclosure is described in more detail below with reference to Examples. However, the present disclosure is not limited to the Examples.

### Example 1

Tables 1 to 3 show vapor-liquid equilibrium data for mixtures of HCC-140, HCO-1130(E), or HCO-1130(Z) with HF at 40°C.

The data demonstrate that azeotropic or azeotrope-like compositions are formed.

**Table 1**

| Liquid phase HF molar ratio | Liquid phase HCC-140 molar ratio | I Gas phase HF molar ratio | Gas phase HCC-140 molar ratio | Total pressure MPa |
|---|---|---|---|---|
| 0.01 | 0.99 | 0.631 | 0.369 | 0.01 7 |
| 0.05 | 0.95 | 0.889 | 0.111 | 0.055 |
| 0.10 | 0.90 | 0.937 | 0.063 | 0.092 |
| 0.20 | 0.80 | 0.963 | 0.037 | 0.146 |
| 0.30 | 0.70 | 0.972 | 0.028 | 0.183 |
| 0.40 | 0.60 | 0.977 | 0.023 | 0.214 |
| 0.50 | 0.50 | 0.978 | 0.022 | 0.214 |
| 0.60 | 0.40 | 0.978 | 0.022 | 0.214 |
| 0.70 | 0.30 | 0.978 | 0.022 | 0.214 |
| 0.80 | 0.20 | 0.978 | 0.022 | 0.214 |
| 0.90 | 0.10 | 0.978 | 0.022 | 0.214 |
| 0.978 | 0.022 | 0.978 | 0.022 | 0.214 |
| 0.99 | 0.01 | 0.978 | 0.022 | 0.214 |
| 0.995 | 0.005 | 0.986 | 0.014 | 0.209 |

**Table 2**

| Liquid phase HF molar ratio | Liquid phase HCO-1130(E) molar ratio | Gas phase HF molar ratio | Gas phase HCO-1130 (E) molar ratio | Total pressure MPa |
|---|---|---|---|---|
| 0.01 | 0.99 | 0.251 | 0.749 | 0.104 |
| 0.05 | 0.95 | 0.576 | 0.424 | 0.181 |
| 0.10 | 0.90 | 0.685 | 0.315 | 0.239 |
| 0.20 | 0.80 | 0.730 | 0.270 | 0.274 |
| 0.27 | 0.73 | 0.730 | 0.270 | 0.274 |
| 0.30 | 0.70 | 0.730 | 0.270 | 0.274 |
| 0.40 | 0.60 | 0.730 | 0.270 | 0.274 |
| 0.50 | 0.50 | 0.730 | 0.270 | 0.274 |
| 0.60 | 0.40 | 0.730 | 0.270 | 0.274 |
| 0.70 | 0.30 | 0.730 | 0.270 | 0.274 |
| 0.80 | 0.20 | 0.730 | 0.270 | 0.274 |
| 0.90 | 0.10 | 0.730 | 0.270 | 0.274 |
| 0.95 | 0.05 | 0.730 | 0.270 | 0.274 |
| 0.99 | 0.01 | 0.770 | 0.230 | 0.260 |

**Table 3**

| Liquid phase HF molar ratio | Liquid phase HCO-1130(Z) molar ratio | Gas phase HF molar ratio | Gas phase HCO-1130 (Z) molar ratio | Total pressure MPa |
|---|---|---|---|---|
| 0.01 | 0.99 | 0.265 | 0.735 | 0.067 |
| 0.05 | 0.95 | 0.609 | 0.391 | 0.125 |
| 0.1 | 0.9 | 0.726 | 0.274 | 0.174 |
| 0.2 | 0.8 | 0.800 | 0.200 | 0.228 |
| 0.26 | 0.74 | 0.817 | 0.183 | 0.244 |
| 0.3 | 0.7 | 0.817 | 0.183 | 0.244 |
| 0.4 | 0.6 | 0.817 | 0.183 | 0.244 |
| 0.5 | 0.5 | 0.817 | 0.183 | 0.244 |
| 0.6 | 0.4 | 0.817 | 0.183 | 0.244 |
| 0.7 | 0.3 | 0.817 | 0.183 | 0.244 |
| 0.8 | 0.2 | 0.817 | 0.183 | 0.244 |
| 0.9 | 0.1 | 0.817 | 0.183 | 0.244 |
| 0.95 | 0.05 | 0.817 | 0.183 | 0.244 |
| 0.99 | 0.01 | 0.829 | 0.171 | 0.241 |

### Example 2 Process of separating 1,1,2-trichloroethane (HCC-140), trans-1,2-dichloroethylene, and cis-1,2-dichloroethylene from hydrogen fluoride

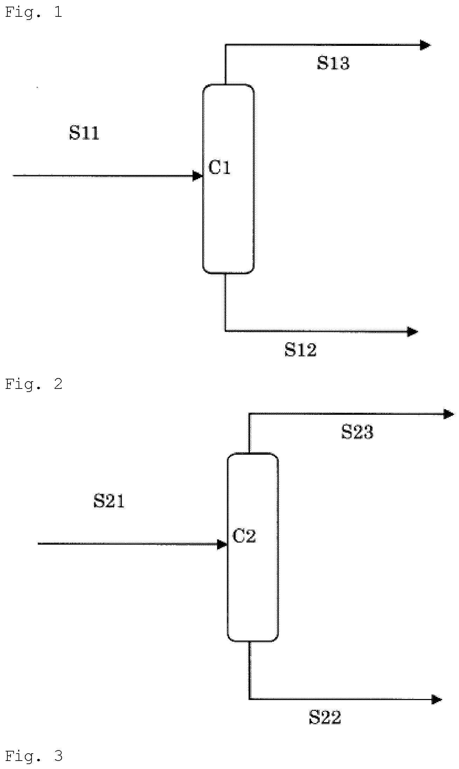

Figs. 1 to 3 show an example of the process of separation by distillation using an azeotropic composition. Fig. 1 and Table 4 show an example of the process of separating HCC-140 from hydrogen fluoride. The unit of the flow rate for each stream is kg/hr. The composition of HCC-140 and hydrogen fluoride from S11 is fed to distillation column C1. HCC-140 with a reduced concentration of hydrogen fluoride is obtained from S12. The stream enriched in hydrogen fluoride, which is obtained from S13, is recycled to the reaction process.

**Table 4**

| | S11 | S12 | S13 |
|---|---|---|---|
| HF | 6.03 | 0.00 | 6.03 |
| HCC-140 | 3.97 | 2.71 | 1.26 |
| Operation pressure | 0.4MPa | | |
| Temperature (°C) | - | 77 | 62 |

Fig. 2 and Table 5 show an example of the process of separating HCO-1130(E) from hydrogen fluoride. The unit of the flow rate for each stream is kg/hr. The composition of HCO-1130(E) and hydrogen fluoride from S21 is fed to distillation column C2. The azeotropic composition of HCO-1130 (E) and hydrogen fluoride is extracted from S23, and hydrogen fluoride in which the concentration of HCO-1130 (E) is reduced is obtained from S22. The stream enriched in hydrogen fluoride, which is obtained from S22, is recycled to the reaction process.

**Table 5**

| | S21 | S22 | S23 |
|---|---|---|---|
| HF | 6.76 | 4.90 | 1.86 |
| HCO-1130(E) | 3.23 | trace | 3.24 |
| Operation pressure | 0.4MPa | | |
| Temperature (°C) | - | 63 | 49 |

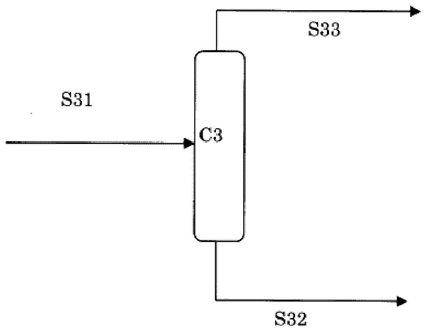

Fig. 3 and Table 6 show an example of the process of separating HCO-1130(Z) from hydrogen fluoride. The unit of the flow rate for each stream is kg/hr. The composition of HCO-1130(Z) and hydrogen fluoride is fed from S31 to distillation column C3. An azeotropic composition of HCO-1130(Z) and hydrogen fluoride is extracted from S33, and hydrogen fluoride with a reduced concentration of HCO-1130(Z) is obtained from S32. The stream enriched in hydrogen fluoride, which is obtained from S32, is recycled to the reaction process.

**Table 6**

| | S31 | S32 | S33 |
|---|---|---|---|
| HF | 6.76 | 3.5 | 3.26 |
| HCO-1130(Z) | 3.24 | trace | 3.24 |
| Operation pressure | 0.4MPa | | |
| Temperature (°C) | - | 63 | 54 |

Regarding the azeotropic compositions of HCC-140 and hydrogen fluoride, HCO-1130(E) and hydrogen fluoride, and HCO-1130(Z) and hydrogen fluoride obtained from S12, S22, and S32, respectively, by reducing the temperature of each composition using a decanter, the solubility of HCC-140, HCO-1130(E), or HCO-1130(Z) relative to hydrogen fluoride can be reduced, which enables obtainment of a composition consisting essentially only of hydrogen fluoride, or further collection of hydrogen fluoride by using absorption of H₂SO₄ etc.

Since the formulation is clarified by making a composition an azeotropic composition, the azeotropic composition can be recycled in a reaction device as is. The clarification of the formulation is beneficial because it facilitates the control of the process.

## Claims

1. An azeotropic or azeotrope-like composition comprising 1,1,2-trichloroethane (HCC-140) and hydrogen fluoride.

2. The azeotropic or azeotrope-like composition according to claim 1, wherein the HCC-140 is present in an amount of more than 10 mass% and 99 mass% or less based on the entire composition.

3. An azeotropic or azeotrope-like composition comprising trans-1,2-dichloroethylene (HCO-1130(E)) and hydrogen fluoride.

4. The azeotropic or azeotrope-like composition according to claim 3, wherein the HCO-1130(E) is present in an amount of more than 10 mass% and 99 mass% or less based on the entire composition.

5. An azeotropic or azeotrope-like composition comprising cis-1,2-dichloroethylene (HCO-1130(Z)) and hydrogen fluoride.

6. The azeotropic or azeotrope-like composition according to claim 5, wherein the HCO-1130(Z) is present in an amount of more than 15 mass% and 99 mass% or less based on the entire composition.

7. A method of separating a mixture comprising HCC-140 and hydrogen fluoride, comprising the steps of:
(a) supplying a composition comprising HCC-140 and hydrogen fluoride to a first distillation column;
(b) extracting, as a first distillate, an azeotropic composition comprising HCC-140 and hydrogen fluoride, and extracting, as a bottom composition of the first distillation column, a composition that is more enriched in either HCC-140 or hydrogen fluoride, in terms of the concentration, than the composition supplied in (a); and optionally
(c) supplying the first distillate to a second distillation column having operation conditions different from the operation conditions of the first distillation column, followed by distillation.

8. A method of separating a mixture comprising HCO-1130(E) and hydrogen fluoride, comprising the steps of:
(d) supplying a composition comprising HCO-1130(E) and hydrogen fluoride to a first distillation column;
(e) extracting, as a first distillate, an azeotropic composition comprising HCO-1130(E) and hydrogen fluoride, and extracting, as a bottom composition of the first distillation column, a composition that is more enriched in either HCO-1130(E) or hydrogen fluoride, in terms of the concentration, than the composition supplied in (d); and optionally
(f) supplying the first distillate to a second distillation column having operation conditions different from the operation conditions of the first distillation column, followed by distillation.

9. A method of separating a mixture comprising HCO-1130(Z) and hydrogen fluoride, comprising the steps of:
(g) supplying a composition comprising HCO-1130(Z) and hydrogen fluoride to a first distillation column;
(h) extracting, as a first distillate, an azeotropic composition comprising HCO-1130(Z) and hydrogen fluoride, and extracting, as a bottom composition of the first distillation column, a composition that is more enriched in either HCO-1130(Z) or hydrogen fluoride, in terms of the concentration, than the composition supplied in (g); and optionally
(i) supplying the first distillate to a second distillation column having operation conditions different from the operation conditions of the first distillation column, followed by distillation.

10. The separation method according to claims 7 to 9, wherein the distillation is performed in a pressure range of 0.05 MPa to 1 MPa.
